# EUROPEAN PATENT APPLICATION

(11) **EP 3 363 894 A1**
(43) Date of publication of application: **22.08.2018**
(21) Application number: 18160689.8
(22) Date of filing: 04.08.2011
(51) Int. Cl.: C12N 7/04, C12N 13/00, A61K 39/25, A61K 39/00

(54) **INACTIVATED VARICELLA ZOSTER VIRUS VACCINES, METHODS OF PRODUCTION, AND USES THEREOF**

(30) Priority: 05.08.2010 US 371038 P
(62) Divisional of application: 11815299.0
(71) Applicant: MERCK SHARP & DOHME CORP., Rahway, NJ 07065-0907 (US)
(72) Inventor: Krah, David L., West Point, Pennsylvania 19486 (US); Dehaven, Jill, Lansdale, Pennsylvania 19446 (US); Kriss, Jennifer A., West Point, Pennsylvania 19486 (US); Barr, Colleen M., West Point, Pennsylvania 19486 (US); Yagodich, Mary, West Point, Pennsylvania 19486 (US)
(74) Representative: Böhles, Elena

(57) **Abstract**

The invention provides an inactivated varicella zoster virus (VZV), and compositions and vaccines comprising said inactivated VZV, wherein the infectivity of the VZV is undetectable and wherein the inactivated VZV induces an immune response against VZV when administered to a patient. In embodiments of the compositions described herein, the VZV is inactivated with gamma radiation. The invention also provides a method of preparing an inactivated VZV vaccine, the method comprising gamma irradiating a sample comprising a VZV using from about 5 kGy to about 50 kGy of gamma radiation. Also provided by the invention herein is a method of treatment of or immunization against HZ or other disease associated with the reactivation of VZV, the method comprising administering to a subject a vaccine or pharmaceutical composition comprising a therapeutically effective amount of an inactivated VZV and a pharmaceutically acceptable carrier, wherein the VZV is inactivated by gamma irradiation.

## Description

### FIELD OF THE INVENTION

The invention relates to compositions and methods for the prevention and treatment of herpes zoster. More specifically, the invention relates to vaccine compositions comprising inactivated varicella virus vaccine (VZV) and methods for producing said compositions.

### BACKGROUND OF THE INVENTION

Primary infection with varicella zoster virus (VZV) causes chicken pox, usually in children and young adults. Although clinical manifestations of chicken pox typically resolve without medical intervention within a short period of time, the VZV can remain latent in sensory neurons for many years following infection. Reactivation and replication of latent VZV, often decades later, can lead to herpes zoster (HZ), commonly known as shingles, a painful rash that is generally limited to a single dermatome. Such VZV reactivation correlates with a decline in cell-mediated immunity, which occurs in the elderly or those who are immunocompromised (Weinberg et al., Journal of Infectious Diseases (2009) 200: 1068-77). In some patients, pain associated with HZ can persist for months or even years after the HZ rash has healed, a complication referred to as post-herpetic neuralgia (PHN).

A live attenuated vaccine (ZOSTAVAX®, Merck & Co., Inc., Whitehouse Station, NJ) is currently available for the prevention of herpes zoster in healthy elderly patients (US Patent Nos. 6,214,354 and 5,997,880). This vaccine has markedly reduced the adverse impacts of HZ in immunocompetent patient populations (Oxman, MN, Clin Infect Dis. (2010) 51(2):197-213; Sanford and Keating, Drugs Aging (2010) 27(2):159-76; Oxman et al., N. Engl. J. Med. (2005) 352: 2271-83). However, live attenuated vaccines may not be suitable for immunocompromised patients.

Immunocompromised individuals, including patients suffering from hematologic malignancy, patients undergoing immunosuppressive therapies, patients who have received a hematopoietic stem cell transplant (HCT) or solid organ transplant (SOT), HIV-infected patients, and patients with autoimmune diseases, have a higher incidence of developing HZ relative to the general population. In addition, these patient populations are at increased risk for developing severe and life-threatening complications (Gourishankar et al. (2004) Am J. Transplant 4: 108-115, Ragozzino et al. (1982) Medicine (Baltimore) 61: 310-316, Wung et al. (2005) Am J Med 118: 1416.e9-1416.e18, Dworkin & Schmader (2003) Clinical Infectious Diseases 36: 877-882, Gebo et al. (2005) J Acquir Immune Defic Syndr 40: 169-174, Mattiuzzi et al. (2003) Clinical Cancer Research 9: 976-980, Dworkin et al. (2003) Neurology 60: 1274-1283.) such as meningoencephalitis (Tauro et al. (2000) Bone Marrow Transplant 26: 795-796), transverse myelitis, visual impairment (Walton et al. (1999) Bone Marrow Transplant 23: 1317-1320), pneumonitis (Wacker et al. (1989) Bone Marrow Transplant 4: 191-194), hepatitis (Rogers et al. (1995) Bone Marrow Transplant 15: 805-807; Schiller et al. (1991) Bone Marrow Transplant 7: 489-491), bacterial superinfection, cutaneous scarring and disfigurement (Schuchter et al. (1989) Blood 74: 1424-1427). Despite the high risk of morbidity and mortality associated with HZ in immunocompromised individuals, this population is not eligible for vaccination with a live attenuated vaccine against HZ such as ZOSTAVAX®. Thus, there is a significant need for a vaccine that would be safe and effective in immunocompromised patient populations to prevent HZ or reduce the severity or duration of HZ or associated complications.

Safety concerns regarding immunocompromised patients have led researchers to investigate the use of non-replicating vaccines against viral pathogens such as subunit vaccines, virus-like particle vaccines and inactivated whole vaccines. Many approved inactivated vaccines contain viruses that are inactivated using formalin, including hepatitis A, polio, and Japanese encephalitis vaccines. For HZ, it has been suggested that a safer vaccine for immunocompromised subjects could be developed through heat inactivation of the VZV or through the use of a subunit vaccine (Cohen, J.I., (2008) J. Infect. Dis. 197(Suppl 2): S237-S241). Redman et al. (J. Infectious Diseases (1997) 176: 578-85) describe an inactivated VZV vaccine which was inactivated by heating at 50°C, resulting in an infectious virus content of ≤ 1.2 pfu/0.5 mL. This level of infectivity would not be desirable for a product to be administered to immunocompromised patients. US Patent Nos. 6,214,354 and 5,997,880 also mention the potential of using an inactivated VZV vaccine and disclose an example of the production and testing of a heat-treated vaccine.

It would meet a significant medical need if a method of inactivating VZV were developed that resulted in a VZV sample that was safe and effective in immunocompromised patients, i.e., lacked residual infectivity but retained the immunogenicity and antigenicity of a non-inactivated sample.

### SUMMARY OF THE INVENTION

It has been shown herein that a VZV sample can be inactivated with gamma irradiation so that the infectivity of the VZV in the sample is at an undetectable level; however, there is no significant loss in immunogenicity and/or antigenicity and no significant change in structure of the VZV upon inactivation by the methods described herein relative to a VZV sample that has not been inactivated.

In one aspect, the invention provides an inactivated varicella zoster virus (VZV), wherein the infectivity of the VZV is undetectable and wherein the inactivated VZV induces an immune response against VZV when administered to a patient. Also provided is a pharmaceutical composition/vaccine comprising a therapeutically effective amount of the inactivated VZV and a pharmaceutically acceptable carrier. The compositions of the invention are either liquid or in a frozen state, e.g. lyophilized. In some embodiments of the compositions described herein, the VZV is inactivated with gamma radiation.

In another aspect, the invention provides a method of preparing an inactivated VZV vaccine, the method comprising gamma irradiating a sample comprising a VZV using from about 5 kGy to about 50 kGy of gamma radiation. In preferred embodiments, the source of gamma radiation is ⁶⁰Co, although other isotopes known in the art may also be useful in this regard. In some embodiments of the method of preparation disclosed herein, the sample is lyophilized prior to irradiating. In alternative embodiments, a liquid bulk is exposed to the gamma radiation, without first being lyophilized.

The invention also provides a method of treatment of or immunization against HZ or other disease associated with reactivation of VZV, the method comprising administering to a subject a vaccine or pharmaceutical composition comprising a therapeutically effective amount of an inactivated VZV and a pharmaceutically acceptable carrier, wherein the VZV is inactivated by gamma irradiation. In some embodiments of the methods of treatment described herein, the route of administration of the composition/vaccine is subcutaneous or intramuscular. In specific embodiments of this aspect of the invention, the patient is 50 years of age or older and/or immunocompromised.

As used throughout the specification and in the appended claims, the singular forms "a," "an," and "the" include the plural reference unless the context clearly dictates otherwise.

As used throughout the specification and appended claims, the following definitions and abbreviations apply:
The term "treatment" refers to both therapeutic treatment and prophylactic or preventative measures. Individuals in need of treatment include those already with the disorder as well as those prone to have the disorder or those in which the disorder is to be prevented. Treatment of a patient with the inactivated VZV of the invention includes one or more of the following: inducing/increasing an immune response against VZV in the patient, preventing, ameliorating, abrogating, or reducing the likelihood of reactivation of VZV in patients who have been infected with varicella or received a live VZV vaccine, preventing or reducing the likelihood of developing HZ and/or other disease or complication associated with VZV reactivation such as PHN, reducing the severity or duration of HZ and/or other disease or complication associated with the reactivation of VZV, such as PHN.

The term "therapeutically effective amount" means a sufficient vaccine composition to produce a desired effect, including, but not limited to: inducing/increasing an immune response against VZV in a patient, preventing, ameliorating or abrogating reactivation of VZV in patients who have been infected with varicella or received a live VZV vaccine, preventing HZ and/or PHN, reducing the severity or duration of HZ and/or PHN. One skilled in the art recognizes that this level may vary.

The term "immune response" refers to a cell-mediated (T-cell) immune response and/or an antibody (B-cell) response.

The term "patient" refers to any human being that is to receive the inactivated VZV vaccines, or pharmaceutical compositions, described herein, including both immunocompetent and immunocompromised individuals. As defined herein, a "patient" includes those already infected with VZV, either through natural infection or vaccination.

The term "bulk" refers to a liquid formulation or composition comprising more than one dose of vaccine.

The term "undetectable levels," in reference to the infectivity of a particular vaccine formulation or composition, means that the formulation or composition comprises <0.050 infectious units or plaque-forming units ("PFU's") of infectious VZV virus per mL of sample, preferably <0.040 PFU's/mL, <0.030 PFU's/mL, <0.020 PFU's/mL, ≤0.015 PFU's/mL, ≤0.010 PFU's/mL, ≤ 0.009 PFU's/mL, or ≤ 0.008 PFU's/mL, more preferably ≤ 0.007 PFU's/mL, ≤ 0.006 PFU's/mL, ≤ 0.005 PFU's/mL, ≤ 0.004 PFU's/mL ≤ 0.003 PFU's/mL, more preferably ≤ 0.002 PFU's/mL, or ≤0.001 PFU's/mL. The PFU's of a particular sample may be determined using, for example, a varicella plaque assay such as the assay described in Example 1 and further described in Krah et al. (J. Virol. Methods (1990) 27: 319-26). The infectivity of a sample may also be confirmed by an immunostaining method, as described in Example 1.

### BRIEF DESCRIPTION OF THE DRAWINGS

Figure 1 shows the inactivation kinetics of γ-irradiated lyophilized VZV (log PFU/ml v. dose of radiation, see Example 4). VZV vials were irradiated with different levels of ⁶⁰Co rays using a ⁶⁰Co Gammacell® irradiator. The residual PFU/mL of each vial per dose of radiation (Gy) was determined using the varicella plaque assay in MRC-5 cells. Observed data points are shown as dark colored diamonds ("observations") and the maximum data measurable by the assay is shown by gray diamonds ("<= Value").
Figure 2 shows inactivation kinetics of γ-irradiated VZV bulk (log pfu/mL titer against radiation time, see Example 4). Different levels of ⁶⁰Co rays were used to irradiate VZV bulk. The irradiated bulk was analyzed for residual infectivity in a varicella plaque assay using MRC-5 cells.
Figures 3A-3F show the VZV response (SFC/10⁶ PBMC) after VZV was inactivated under different conditions as measured by the VZV IFNγ ELISPOT assay (see Example 5). Data are shown for 6 donor PBMC samples (figures A-F) for five heat-treated VZV bulk preparations and two gamma-irradiated VZV bulk preparations. Also shown are the VZV responses for live (untreated) VZV samples from the same bulk preparations as those used above and a lot of VZV that was inactivated by treatment with UV light (VZV lot # 96.07).
Figures 4A-4C show pictures of VZV viral particles, as assessed by cryo EM (See Example 6). Shown are lyophilized samples that were untreated (Figure 4A), irradiated with 25 kGy (Figure 4B) and irradiated with 50 kGy (Figure 4C).
Figure 5 shows the results of a mouse immunogenicity study in which the VZV titer (IgG response) for a set of inactivated VZV preparations generated using various methods for inactivation (see Example 8) were evaluated in the VZV ELISA assay. The adjusted VZV titers (VZV titer minus MRC-5 titer) are shown for each mouse, along with the geometric mean titer. Also shown are the results from 2 mice in the heat-treated lyophilized group that were considered to be outliers.
Figure 6 shows the statistical analysis of VZV gpELISA antibody responses by time points among human vaccine recipients who received either heat-treated VZV vaccine (N=65) or gamma-irradiated VZV vaccine (N=63) as described in Example 9.
Figure 7 shows the statistical analysis of VZV gpELISA antibody responses by time points among human vaccine recipients who received either gamma irradiated VZV vaccine B (16-25 kGy, N=64) or gamma irradiated vaccine C (25-50 kGy, N=65), as described in Example 10.

### DETAILED DESCRIPTION OF THE INVENTION

It has been shown herein that a bulk or lyophilized VZV sample can be inactivated with gamma irradiation so that the infectivity of the VZV in the sample is at an undetectable level (for example, the infectivity of inactivated VZV samples described herein was ≤0.001 PFUs/mL). The inactivated VZV produced by the methods described herein can be formulated with a pharmaceutically acceptable carrier to produce a pharmaceutical composition or vaccine for use in methods of treatment/immunization of the invention. There is no significant loss in immunogenicity and/or antigenicity and no significant change in structure of the VZV upon inactivation by the methods described herein relative to a VZV sample that has not been inactivated. Thus, when the pharmaceutical compositions of the invention are administered to a patient, the immune response elicited by the composition is not significantly different than the immune response elicited by a control sample comprising the same amount of the VZV that has not been inactivated. As used herein, an immune response elicited by an inactivated sample that is "not significantly different" from a non-inactivated control sample differs from the control sample by about 50% or less, more preferably about 40 % or less, about 30 % or less, even more preferably about 20% or less, 15% or less, 10% or less, or 5% or less.

The immune response elicited by an inactivated and a control sample may be measured in an appropriate animal model or human population in a clinical trial, for example, by measuring one or more of the following parameters: (1) the VZV specific responder cell frequency (as described in Calandra et al., WO 94/002596, (2) the anti-VZV cytotoxic T-cells (CTL's), or VZV specific CD8⁺ cells, (3) the anti-VZV helper T-cells, or VZV specific CD4⁺ cells, (4) the level of anti-VZV specific antibodies, or (5) the level of lymphokines such as interferon, or interleukin.

Techniques that are useful for measuring the immune response are known in the art and include, but are not limited to: a T-cell response assay, a potency assay, a VZV IFNγ ELISPOT assay, and an ELISA assay.

To determine the sufficiency of the immune response elicited by the vaccines/ pharmaceutical compositions of the present invention and made by the methods described herein, and also the efficacy of the inactivated vaccine, it may be useful to measure clinical outcomes in a clinical trial of human volunteers/subjects to measure, for example, a reduction of the duration or severity of HZ and/or a reduction in the duration of PHN in an individual to a period of less than one month following development of zoster, reduction in the incidence of zoster in the patient population, on a statistical level, below the incidence found in the general population or of similarly at-risk or immunocompromised individuals.

As stated above, the present invention shows that VZV preparations can be inactivated to undetectable levels, while still retaining the antigencity and immunogenicity of a similar non-inactivated VZV. Accordingly, one aspect of the invention provides an inactivated VZV, wherein the infectivity of the VZV is undetectable and wherein the inactivated VZV induces an immune response against VZV when administered to a patient.

One skilled in the art can readily determine a proper amount of VZV antigen to be used as a therapeutically effective amount of VZV. Such amount will vary according to intended use, or other factors such as the particular patient population. In some embodiments of the methods and compositions described herein, the amount of VZV is from about 1 to about 10 VZV antigen units/dose, with 1 VZV antigen unit being roughly equivalent to 1 µg of purified VZV protein. In specific embodiments, the amount of VZV antigen present in a composition is between about 2 and about 8 VZV Ag units/dose or between about 3 and about 6 VZV Ag units/dose. The amount of antigen can be determined with an appropriate antigen assay, for example, the competitive ELISA described in Example 2 herein. One skilled in the art can determine other appropriate assays to measure the amount of antigen in a sample.

Also provided herein is a pharmaceutical composition comprising a therapeutically effective amount of the inactivated VZV described above and a pharmaceutically acceptable carrier, excipient or diluent. Pharmaceutically acceptable carriers useful in the compositions of the invention include any compatible agent that is nontoxic to patients at the dosages and concentrations employed, such as water, saline, dextrose, glycerol, ethanol, buffers, and the like, and combinations thereof. The carrier may also contain additional components such as a stabilizer, a solubilizer, a tonicity modifier, such as NaCl, MgCl₂, or CaCl₂ etc., a surfactant, and mixtures thereof.

The invention also provides multi-dose compositions which contain more than one dose of the inactivated VZV, an anti-microbial preservative, which prevents inadvertent microbial contamination upon introduction of a syringe to the vial comprising the composition, and a pharmaceutically acceptable carrier. Such multi-dose compositions can be provided to a patient more than one time, after a predetermined amount of time has passed or to more than one patient.

In some embodiments of the invention, the composition is a liquid bulk which contains more than one dose of inactivated VZV. In alternative embodiments, the composition is lyophilized. Methods of lyophilization are known in the art. Also encompassed by the invention is a lyophilized formulation that is reconstituted with an appropriate diluent such as sterile water for injection or bacteriostatic water for injection.

Gamma irradiation is commonly used to sterilize devices and equipment for use in medical applications. It is also used to kill any potential pathogens in biologicals, such as blood products and antibody preparations. For these uses, an organism is inactivated without regard to maintaining the function of the organism. There are limited literature sources that describe the inactivation of viruses by gamma irradiation.

Rosen et al. (Int. J. Radiat. Biol. 52:795-804 (1987)) evaluated herpes simplex virus intact virus particles for survival of plaque-forming ability and purified genomes for damage to DNA molecules after treatment with ⁶⁰Co rays. This study did not evaluate the antigenicity or immunogenicity of the virus after irradiation. Another study by Elliott et al. (J. Clin. Micobiol. 16: 704-708 (1982)) evaluated the inactivation of Lassa, Marburg, and Ebola viruses using ⁶⁰Co rays. Linear kinetics of inactivation were observed for Lassa, Ebola, and Marburg viruses and virus inactivation was achieved by gamma irradiation using ⁶⁰Co rays. Elliott *et al.* observed that a greater dose of radiation was required for inactivation of the virus in the frozen state than the liquid state. Like Rosen *et al.,* this study failed to determine the antigenicity or immunogenicity of the viruses after gamma irradiation. Alsharifi et al. (WO 2010/012045) describe the use of gamma irradiation to inactivate influenza virus, an RNA virus from the family Orthomyxoviridae, for use as a vaccine.

In the invention described herein, it is shown that gamma radiation can reduce the infectivity of a VZV viral preparation to undetectable levels, while retaining the antigenicity, immunogenicity and structural characteristics of an non-inactivated control. Thus, in preferred embodiments of the invention, the VZV is inactivated by gamma irradiation, which can be delivered to the VZV by exposure to an appropriate isotope such as ⁶⁰Co, ¹³⁷Cesium, ⁹⁹Technetium or ^{99m}Tc. In preferred embodiments, the gamma radiation is delivered to the VZV by exposure to ⁶⁰Co rays.

The amount of gamma radiation useful for inactivating the VZV is from about 5 to about 50 kiloGrays (kGy) of gamma radiation. A Gray (Gy) is a standard unit of absorbed dose, with one gray being equal to an absorbed dose of 1 Joule/kilogram (100 rads). One skilled in the art will realize that the amount of radiation exposed to the sample and amount of time should be varied in order to reach a desired absorbed dose of radiation (dose = fluence x time). One skilled in the art will also be able to vary the amount of radiation that the sample is exposed to depending on the size of the container within which the sample is being stored so that the proper dose of radiation is delivered to the entire sample without over-exposing a portion of the sample, for example, the portion closest to the radioactive source.

In some embodiments of the methods and compositions of the invention described herein, the amount of gamma radiation used to inactivate the VZV is about 25 kGy or less. In alternative embodiments, the amount of gamma radiation is in the range of about 5 kGy to about 40 kGy, about 5 kGy to about 35 kGy, about 5 kGy to about 30 kGy, about 5 kGy to about 25 kGy, about 5 kGy to about 20 kGy, about 5 kGy to about 10 kGy, about 10 kGy to about 50 kGy, 10 kGy to about 40 kGy, about 10 kGy to about 35 kGy, about 10 kGy to about 30 kGy, about 10 kGy to about 25 kGy, about 10 kGy to about 20 kGy, about 15 kGy to about 50 kGy, about 15 kGy to about 40 kGy, about 15 kGy to about 35 kGy, about 15 kGy to about 30 kGy, about 15 kGy to about 25 kGy, about 15 kGy to about 20 kGy.

The invention also provides a pharmaceutical composition as described above, and methods of producing said composition, wherein the composition is a liquid bulk and the amount of gamma radiation used to inactivate the VZV is from about 5 kGy to about 10 kGy or from about 5 kGy to about 12.5 kGy. In other specific embodiments, the composition is lyophilized prior to gamma irradiation and in such embodiment, the amount of gamma radiation is from about 5 kGy to about 25 kGy or from about 15 kGy to about 25 kGy.

It is shown herein that linear inactivation kinetics were observed for VZV inactivated with gamma radiation, which is of significant benefit in allowing modeling of inactivation, i.e. reliably estimating the level of residual infectivity in a sample comprising inactivated VZV. Such linear inactivation kinetics were not observed for heat treated inactivated VZV. Grieb et al. (Biologicals (2002) 30:207-216 and Biomaterials (2005) 26:2033-2042) described two specific mechanisms for the inactivation of viruses by gamma irradiation, which support the linear inactivation kinetics observed for VZV. The first mechanism is a "direct result of a photon depositing energy into the target". This direct energy transfer results "in the dislocation of outer electrons from molecules and breakage of covalent bonds." The second, "indirect", mechanism is a result of a chemical attack on free radicals and reactive oxygen species typically generated by the interaction of radiation with water molecules and oxygen. In specific embodiments of the compositions and methods described herein, the VZV is lyophilized prior to inactivation with gamma radiation; thus, there is unlikely to be much interaction with water molecules because water is removed from the product in the lyophilization process.

Redman et al. (J. Infectious Diseases (1997) 176: 578-85) describe an inactivated VZV vaccine which was inactivated by heating to 50°C, resulting in an infectious virus content of ≤ 1.2 pfu/0.5 mL. The heat-treated vaccine described by Redman and colleagues was shown to reduce disease severity associated with VZV reactivation among 24 patients scheduled to undergo autologous BMT or peripheral blood stem-cell infusion or other BMT. This level of infectivity is not suitable for all patients, such as those who are immunocompromised. In addition, methods of inactivation of VZV using heat treatment require lengthy periods of time to reach low levels of infectivity, which is inefficient and not cost-effective.

To that end, the invention provides inactivated VZV and pharmaceutical compositions/vaccines comprising said inactivated VZV, wherein the infectivity of the VZV is at an undetectable level, i.e. <0.050 PFU's/mL, said inactivated VZV compositions being safer than previously disclosed vaccines for the treatment and/or prevention of HZ or disease associated with VZV reactivation and suitable to a more efficient manufacturing process. The inactivated VZVs of the present invention retain the structural physical characteristics, immunogenicity and antigenicty of a non-inactivated VZV control following inactivation. In some embodiments, the infectivity is <0.040 PFU's/mL, <0.030 PFU's/mL, or <0.020 PFU's/mL. The invention also provides embodiments wherein the number of infectious units is ≤0.015 PFU's/mL, ≤0.010 PFU's/mL, ≤ 0.009 PFU's/mL, or ≤ 0.008 PFU's/mL. In alternative embodiments, the infectivity of the VZV is ≤ 0.007 PFU's/mL, ≤ 0.006 PFU's/mL, ≤ 0.005 PFU's/mL, ≤ 0.004 PFU's/mL or ≤ 0.003 PFU's/mL. In additional alternative embodiments, the infectivity is ≤ 0.002 PFU's/mL or ≤0.001 PFU's/mL.

In some embodiments of the compositions and methods provided herein, the infectivity (PFU's) of the sample is determined using a varicella plaque assay such as the assay described in Example 1 and further described in Krah et al. (J. Virol. Methods (1990) 27: 319-26). One skilled in the art will realize that other methods are also useful for determining infectivity of the VZV sample or composition and may be used in place of the varicella plaque assay.

Any VZV strain can be used in the compositions and methods described herein, including a wild type varicella strain, or an attenuated strain such as the Oka strain, as described in U.S. Patent 3,985,615 and available from the American Type Culture Collection (Accession no. VR-795™, ATCC, Manassas, VA). The Oka strain was originally obtained from a healthy boy with a natural varicella infection and passaged in human embryonic lung cells. It is adapted to growth in guinea pig embryo cell cultures and human diploid lung fibroblast cell cultures (e. g., MRC-5 cells). In preferred embodiments of the compositions and methods described herein, the VZV is an Oka strain or an Oka strain derivative, such as the Oka/Merck VZV strain. An "Oka strain derivative," as used herein, is a strain that is obtained by a process of further passaging the Oka strain in an appropriate cell type in order to sufficiently attenuate the strain so as to be useful, for example, as a live attenuated vaccine. In the methods and compositions of the invention described herein, a live or attenuated VZV, such as the Oka strain or derivative thereof, is inactivated with gamma radiation prior to administration to a patient.

Also provided herein is a method of preparing an inactivated VZV comprising gamma irradiating a sample comprising a VZV using from about 5 to about 50 kGy of gamma radiation. In preferred embodiments of this aspect of the invention, gamma radiation is provided to the sample by exposing the sample to ⁶⁰Co rays. In particular embodiments of this aspect of the invention, the amount of gamma radiation is as discussed, *supra.*

The invention also provides an inactivated VZV produced or obtainable by the methods described herein. Additionally provided is a vaccine comprising a therapeutically effective amount of the inactivated VZV produced by the methods described herein and a pharmaceutically acceptable carrier.

The invention also provides, in one aspect, a method for the treatment, prevention of, immunization against, or reduction in the likelihood of herpes zoster and/or other disease or complication associated with the reactivation of VZV, e.g., post-herpetic neuralgia, in a patient, the method comprising administering to the patient a therapeutically effective amount of a vaccine or pharmaceutical composition comprising an inactivated VZV and a pharmaceutically acceptable carrier, wherein the VZV is inactivated with gamma irradiation. Since VZV reactivation correlates with a decline in cell-mediated immunity, the methods of treatment herein are useful to boost the cell-mediated immune response in a patient that was previously exposed to varicella through natural infection or vaccination, but whose immune response has declined as a result of advancing age and/or immune system dysfunction.

In the methods of treatment/immunization described above, the pharmaceutical composition comprising an inactivated VZV may be administered to the patient through any suitable route including, but not limited to: subcutaneous injection, intradermal introduction, impression though the skin, or other modes of administration such as, intravenous, intramuscular or inhalation delivery. In preferred embodiments of the methods described herein, the mode of administration is subcutaneous or intramuscular.

The methods and compositions described above are useful for preventing HZ and/or PHN, or reducing the severity or duration thereof in immunocompetent and immunocompromised patient populations including, but not limited to, healthy patients and immunocompromised patients who have undergone hematopoietic stem cell transplant (HCT) or solid organ transplant (SOT), HIV-infected patients, patients with autoimmune diseases, individuals with blood cancers; individuals receiving chemotherapy across a broad range of solid tumors malignancies; patients receiving chronic immunosuppressive therapy across a broad range of conditions including rheumatoid arthritis (RA), systemic lupus (SLE), Crohn's disease, psoriasis, and multiple sclerosis. In some embodiments of the methods described herein, the inactivated VZV vaccine is administered to a patient who is at greater risk for HZ due to disease, for example, the diseases mentioned above, or treatment for disease (such as hematologic malignancies, solid tumor malignancies or chemotherapy, autoimmune diseases).

In some embodiments of any of the methods and compositions described above, the patient is 50 years of age or older and may be healthy or immunocompromised. In other embodiments, the patient is 55 years of age or older, 60 years of age or older, 65 years of age or older, 70 years of age or older, or 75 years of age or older. In alternative embodiments, the patient is from about 50 to about 55 years of age, from about 55 to about 60 years of age, from about 60 to about 65 years of age, or from about 65 to 70 years of age.

In additional embodiments of the methods described herein, the vaccine or pharmaceutical composition is administered concominantly with other commonly administered 'standard of care' therapies; or with other vaccines for targeted patient populations, including, for example, a pneumococcal vaccine such as PNEUMOVAX™ 23 (PN23, Merck & Co., Inc.) a hepatitis B (HBV) vaccine such as RECOMBIVAX™ HB or ENGERIX-B™ (GlaxoSmithKline Biologicals) and flu vaccines.

In specific embodiments of the methods of treatment/prevention provided herein, the method further comprises allowing an appropriate predetermined amount of time to pass and administering to the patient one or more additional doses of the pharmaceutical composition. In said embodiments, one additional dose may be administered to the patient after an appropriate amount of time has passed, alternatively, two, three or four additional doses, each being administered after an appropriate amount of time has passed. In an exemplary embodiment, 3 or 4 doses are administered to the patient as part of a dosing regimen that is properly separated over a course of time. One skilled in the art will realize that the amount of time between doses may vary depending on the patient population, dosage of the vaccine and/or patient compliance. In an exemplary embodiment, a time period of about 3 weeks, about 1 month, about 6 weeks, about 2 months, or about 3 months or more is allowed to pass between administrations of each dose to the patient. In one specific embodiment the vaccine is administered to a patient that is to undergo a transplant before the transplant, e.g. 3 weeks, 1 month or 2 months, pre-transplant, and an additional one or more doses are given to the patient at an appropriate time after the transplant, e.g. 3 weeks, 1 month or 2 months post-transplant, with a predetermined amount of time passing between each dose.

All publications mentioned herein are incorporated by reference for the purpose of describing and disclosing methodologies and materials that might be used in connection with the present invention. Nothing herein is to be construed as an admission that the invention is not entitled to antedate such disclosure by virtue of prior invention.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

The following examples illustrate, but do not limit the invention.

### Materials and Methods

### EXAMPLE 1

### Varicella Plaque Assay

The infectivity titers of varicella zoster virus (VZV) preparations were measured using the liquid overlay procedure described by Krah et al. (J. Virol Methods (1990) 27: 319-326). The assay was performed as follows: MRC-5 (human diploid lung fibroblast) cells were seeded in 60-mm tissue culture plates at 600,000 cells in 5 mL volumes of BME (Basal Medium Eagle with Hanks' balanced salts solution) with 100 mg/L galactose, 50 ug/mL neomycin, 2 mM L-glutamine, and were incubated at 35± 1°C in a 5% ± 1% CO₂ humidified atmosphere. After incubating for 24-56 hours, the cells reached 50-80% confluency and were used for the plaque assay. The cell growth medium was removed by aspiration, and the cells were infected with 100 µL aliquots of VZV solution diluted in appropriate diluent, such as PGS stabilizer (PBS with sucrose and hydrolyzed gelatin).

Virus was allowed to attach for ≥1 hour at 35± 1°C in a 5% ± 1% CO₂ humidified atmosphere. The cultures were then overlaid with 5 mL volumes of maintenance medium (Minimal Essential Medium with Earle's salts (MEM), 2% heat-inactivated fetal calf serum, 50 µg/mL neomycin and 2 mM L-glutamine), and returned to incubation at 35± 1°C in a 5% ± 1% CO₂ humidified atmosphere for 6-7 days for plaque development. Medium was then aspirated from the plates and plaques were visualized by staining cells with a solution of 0.2% (w/v) Coomassie Blue R-250 in ethanol, 1% acetic acid. Plaque counts were the average of 3-5 replicate plates, and were multiplied by the dilution factor (the reciprocal of the corresponding dilution of virus tested) and the inoculum volume correction factor (10, to adjust from 0.1 mL to 1 mL) to provide plaque-forming units/mL (PFU/mL).

Given the critical need to assure the absence of false-positive PFU results for inactivated samples (where a plaque-like spot may appear in the cell monolayer used for the plaque assay due to inadvertent scratching of the monolayer or due to the presence of a clump of cells), an immunostaining method was developed and applied to verify that foci that were counted as plaques were indeed VZV-infected foci of cells (VZV plaques). Briefly, following the staining with Coomassie Blue in the standard plaque assay, cultures with visible plaques were incubated with a polyclonal anti-VZV serum diluted in PBS, 0.05% Tween 20 and incubated for 1 hour at 35°C. Cells were washed with PBS, 0.05% Tween 20 to remove unbound antibody and bound antibody was detected using a peroxidase-conjugated goat anti-human IgG antibody and a peroxidase substrate (diaminobenzidine solutuion) that develops a precipitate when reacted with peroxidase. A brown precipitate developed around the foci containing the VZV, and no additional plaques beyond those observed using the Coomassie Blue stain were detected. Therefore this immunostaining method, using a combination of anti-VZV primary serum, peroxidase-conjugated anti-primary species antibody and peroxiadse substrate, provides a method to verify whether a suspected plaque in an inactivated sample is indeed represents residual infectious VZV.

### EXAMPLE 2

### Competitive VZV Antigen ELISA and Direct Binding VZV Antigen ELISA Assays

Two antigen assay formats were used to characterize and quantify VZV antigen. A competitive antigen ELISA, which mimics the test used to estimate VZV antigen in VZV commercial product and utilizes a single polyclonal serum to detect VZV antigen, was used in select studies. To provide additional characterization of the reactivity of VZV antigen following gamma irradiation or other inactivation treatments, a direct binding ELISA was developed and applied, wherein the test sample was directly adsorbed to microtiter ELISA plates and then detected using monoclonal or polyclonal antibodies.

### Competitive ELISA for Quantitation of VZV Antigen

Briefly, this assay was conducted by incubation of VZV antigen from test samples with a fixed amount of polyclonal anti-VZV serum in solution. Remaining free antibody (not bound to antigen) was allowed to bind to a standard VZV antigen immobilized on ELISA microtiter plates. The amount of antibody capable of binding to the plates is inversely proportional to the amount of antigen in the test sample. Antibody binding to the plates was quantitated by reaction with an enzyme-linked anti-human antibody and appropriate substrate to provide a colored product which was quantitated spectrophotometrically.

The test procedure comprised the following steps: (1) ELISA plates were coated with glycoproteins (gps) from VZV-infected or uninfected MRC-5 cells, and were overcoated with 1% (w/v) bovine serum albumin to reduce non-specific antibody adsorption to the plates. (2) Test VZV antigens were diluted in PGS stabilizer in polypropylene microtubes. A VZV antigen preparation of known antigen titer was included as a control. Samples were typically diluted through serial 1:1.25-fold dilutions to include antigen concentrations ranging from 2.6 to 0.9 antigen units/mL. The dilution series of the VZV standard was used to generate a standard curve for the measurement of antigen in the test samples. (3) A human anti-VZV serum was diluted in PGS stabilizer to two-times the desired final dilution (4) Three hundred µL volumes of diluted antigen were dispensed into polypropylene microtubes, mixed with 300 µL of diluted anti-VZV serum and incubated at 35-37°C for 15-30 min. The anti-VZV serum plus diluent (no antigen) was used as a control. (5) Aliquots of 100 µL from each serum-antigen mixture (and control) were added to 2 replicate VZV gp coated wells and 2 MRC-5 gp coated wells. (6) Plates were incubated for 15-30 min at 35-37°C to allow free antibody (not complexed to test antigen in solution) to bind to the gp antigen immobilized on the plates. (7) Unbound antibody was removed by washing and wells received an alkaline phosphatase-conjugated goat anti-human IgG antibody solution to detect bound human antibody. (8) After incubation for 15-30 min at 35-37°C, unbound conjugate was removed by washing. Bound conjugate was detected by incubation for 10-15 min at 35-37°C with p-nitrophenyl phosphate substrate. (9) After termination of the substrate reaction by addition of 50 µL 3 M NaOH, color development (OD at 405 nm) was quantitated using a microplate spectrophotometer.

Test calculation and interpretation comprised the following steps: (1) Respective replicate OD values for the replicate VZV and MRC-5 gp coated wells were averaged. It was known from experience that the MRC-5 OD is consistent between different samples and dilutions. Therefore, the MRC-5 gp OD values for the entire set tested were typically averaged and used to correct for non-specific binding of the primary antibody (the anti-VZV serum). The averaged MRV-5 gp OD was subtracted from the respective averaged VZV gp ODs to provide VZV specific (ΔOD) values. (2) Generation of a standard curve for measurement of antigen amounts: The standard curve ΔOD values were plotted against the known antigen concentrations (VZV antigen units/mL). The data were entered into an appropriate graphics program, the linear portion of the curve was identified and the "line fit formula" (y=a+bx) for this linear curve was obtained. (3) Calculation of antigen amounts of test samples: Because values for a and b are given by the line-fit formula, and y (ΔOD) is known, the unknown value, x, representing the units/mL antigen, could then be calculated and corrected by the sample dilution to obtain the antigen concentration of the original (undiluted) test sample. The reported antigen concentration is that obtained with the least diluted sample providing a ΔOD value within the linear portion of the standard curve.

### Direct Binding VZV Antigen ELISA

Briefly, this assay was conducted by incubation of dilutions of VZV antigen from test samples in 96-well microtiter plates to immobilize the antigen, followed by detection of the bound antigen with monoclonal or polyclonal anti-VZV antibodies. Bound anti-VZV antibodies were quantitated by reaction with an enzyme-linked anti-appropriate-species antibody and appropriate substrate to provide a colored product which was quantitated spectrophotometrically. Titration curves of OD versus dilution for the samples were then compared (quantitated as the fold-dilution difference between titration curves ("curve shift").

The test procedure comprised the following steps: (1) High-binding ELISA plates were coated with 200µl antigen (vaccine) diluted in PBS 1:20 through 1:2560 in serial 2-fold dilutions. The plates were covered and stored @ 2-8°C overnight. (2) Liquid from step #1 was poured off and plates were rinsed 3 times with PBS + 0.05% Tween 20. The final rinse was removed and diluted antibody (100 µl/well) was added. Plates were incubated for 1 hour @ 35 °C. (3) Liquid from step #2 was poured off and the plates were rinsed 3 times with PBS + 0.05% Tween 20. The final rinse was removed and diluted conjugate (100 µl/well) was added. Plates were incubated for 1 hour @ 35°C. (4) Liquid from step #3 was poured off and the plates were rinsed 3 times with PBS + 0.05% Tween 20. The final rinse was removed and substrate (undiluted) was added. Plates were then incubated for 30min @ 35°C. (5) The reaction was stopped with NaOH. (6) Optical density (OD) was determined by reading the plate at 405nm.

Antibodies used for this experiment were as follows: EPP serum and purified monoclonal antibodies to gE (Biodesign catalog # mab8612), gB (Chemicon catalog #C05102M, Millipore Corp., Billerica, MA), and gH (Biodesign cat#C05104M and Virusys catalog #VA033-100, Virusys Corp., Taneytown, MD). Conjugates used were either anti-human IgG (BioSource catalog # AHI0305, Invitrogen Corp., Carlsbad, CA) or goat anti-mouse IgG (Pierce catalog # 31322, Pierce Biotechnology Inc., Rockford, IL). Alkaline phosphatase pNPP (Sigma cat#p7988, Sigma-Aldrich Co., St. Louis, MO) 100mL was used as substrate.

### EXAMPLE 3

### VZV IFNγ ELISPOT Assay

Briefly, serial dilutions of test antigens, ranging from 1 to 0.125 VZV antigen units/mL, were incubated with PBMC from 6 donors in duplicate wells. Following incubation, the cells responding to VZV antigen were identified by their ability to produce interferon gamma. Interferon gamma production was detected using mouse anti-interferon gamma coated on a microtiter plate, and a matched secondary anti-mouse IgG enzyme conjugated antibody and substrate, leading to detection of precipitated substrate around the interferon gamma producing cells ("spots"). The number of spot forming cells (SFC: those that form spots indicating interferon gamma secretion) was quantitated by an automated ELISPOT reader, and results were expressed as SFC per 10⁶ PBMC.

### Results

### EXAMPLE 4

### Inactivation Kinetics of Gamma Irradiated VZV.

We had previously tested heat treatment as a method of inactivating lyophilized VZV for use as a vaccine for immunocompromised patients, but found that this method resulted in residual infectivity of the viral preparation (see below). It was considered that this residual infectivity was not an optimum characteristic of the "inactivated" vaccine. More extensive inactivation of liquid bulk vaccine was achieved relative to lyophilized vaccine, but heat-treatment of bulk vaccine was determined to not provide an optimum manufacturing process. Thus, an alternate inactivation method for varicella-zoster virus vaccine (Oka/Merck) lyophilized using gamma irradiation ⁶⁰Co was investigated to see if a more extensive inactivation of infectivity (yielding a more favorable safer profile) method than heat treatment could be identified. The goals of this evaluation were first to determine if more extensive inactivation of infectivity could be achieved by ⁶⁰Co irradiation, and then secondly, whether the antigen properties achieved following gamma irradiation differed from those of heat-treated material and were acceptable for clinical application studies. VZV vials were irradiated using ∼1, 2, 3, 4, 5, 6, 7, 8 kGy (2007) or 0.45, 1.35, and 2.7 kGy. Irradiation was performed using a ⁶⁰Co Gammacell® irradiator (Best Theratronics Ltd. Corporation, Ottawa, Ontario, Canada).

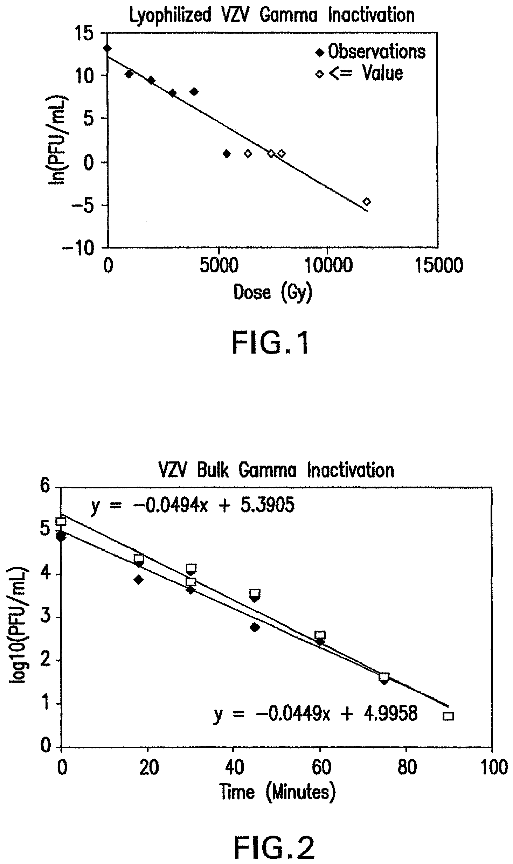

The residual PFU/mL of each vial per dose of radiation (Gy) was determined using the varicella plaque assay in MRC-5 cells. The log PFU/mL was plotted against the dose of radiation and the data points showed a linear regression of inactivation (FIGURE 1). Following the kinetic study, we tested 100 mL of inactivated VZV vaccine irradiated with ⁶⁰Co rays to 12kGy (inactivated lyophilized) to permit extended confirmation of the extent of inactivation (by testing a larger total volume). Zero plaques were detected in the 100 mL sample.

Additionally, ⁶⁰Co rays were used to irradiate VZV bulk. The bulk was thawed and aliquoted in PETG bottles for irradiation at 0.5, 1, 2, 3, 4, 5, 6 kGy (FIGURE 2). The irradiated bulk was analyzed for residual infectivity in a varicella plaque assay using MRC-5 cells. Zero plaques were detected in the 100 mL samples of irradiated bulk (6 kGy). Linear inactivation kinetics were observed by plotting the log pfu/mL titer against radiation time.

### EXAMPLE 5

### Evaluation of Immunogenicity by VZV IFNγ ELISPOT Assay

To evaluate the impact of different inactivation methods on immunogenicity, a VZV IFNγ ELISPOT assay was performed as described in Example 3 using 6 donor peripheral blood mononuclear cell (PBMC) samples. This study was conducted to determine if the IFN-γ response diminishes after VZV is inactivated under different conditions. Five heat-treated VZV bulk preparations were tested, along with two gamma-irradiated VZV bulk preparations. The heat treated VZV preparations consisted of samples that were treated under the following conditions: (1) 40°C for 24 hours, (2) 45°C for 3.5 hours, (3) 45°C for 4.5 hours, (4) 56°C for 30 minutes, and (5) 56°C for 90 minutes. The gamma-irradiated samples consisted of VZV bulks gamma-irradiated for 3 or 4 hours at 5.92 kGy. Live (untreated) VZV samples from the same bulk preparations as those used above to create inactivated samples were also tested for comparison.

A lot of VZV that was inactivated by treatment with UV light was also included as an ELISPOT antigen (VZV lot # 96.07) as an assay run control along with PHA (positive control) and media (negative control). All antigens were evaluated across a serial two-fold dilution from 1:40 to 1:320. One assay run was performed testing 6 PBMC samples, chosen to represent a range of VZV response levels.

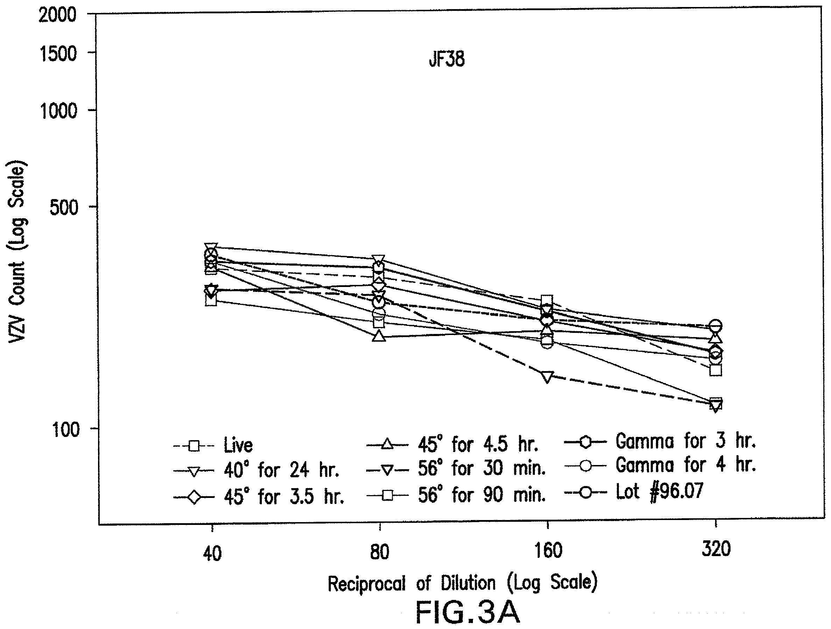

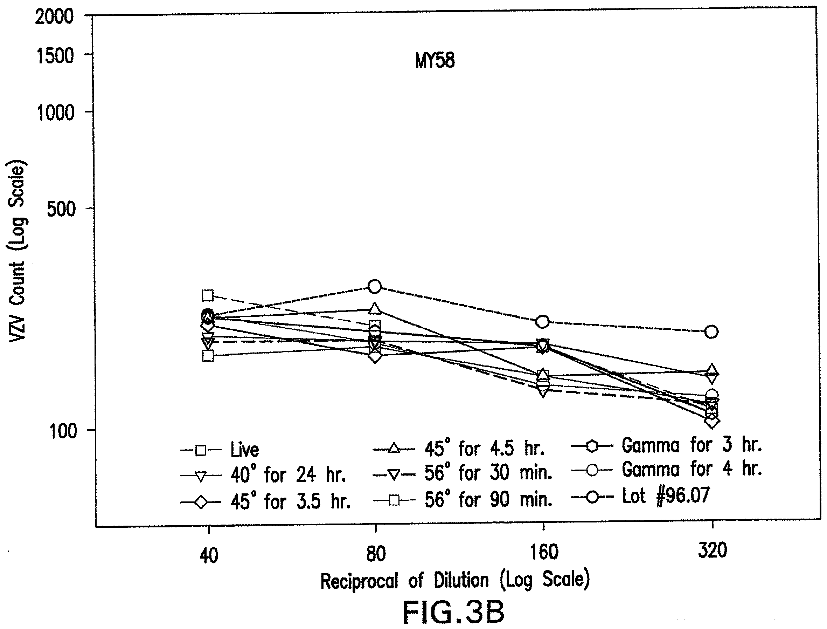

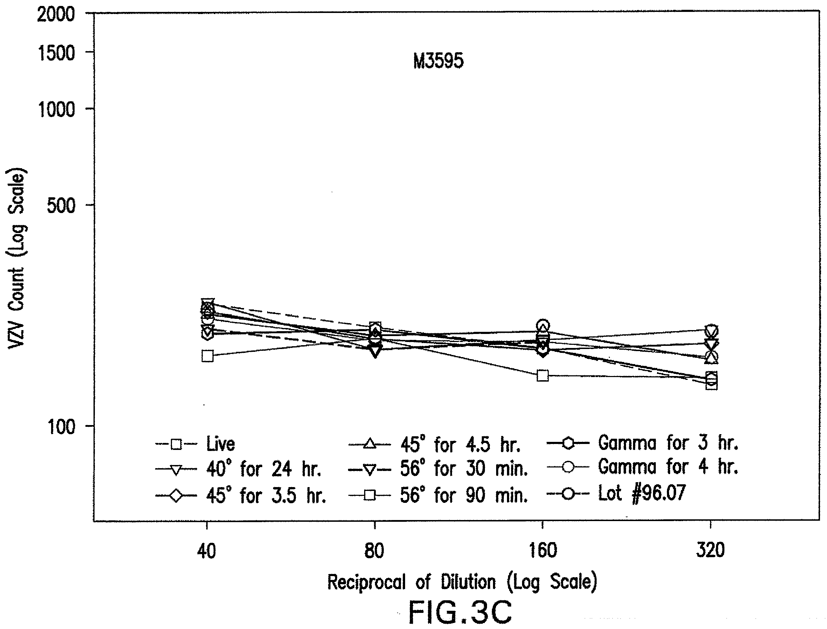

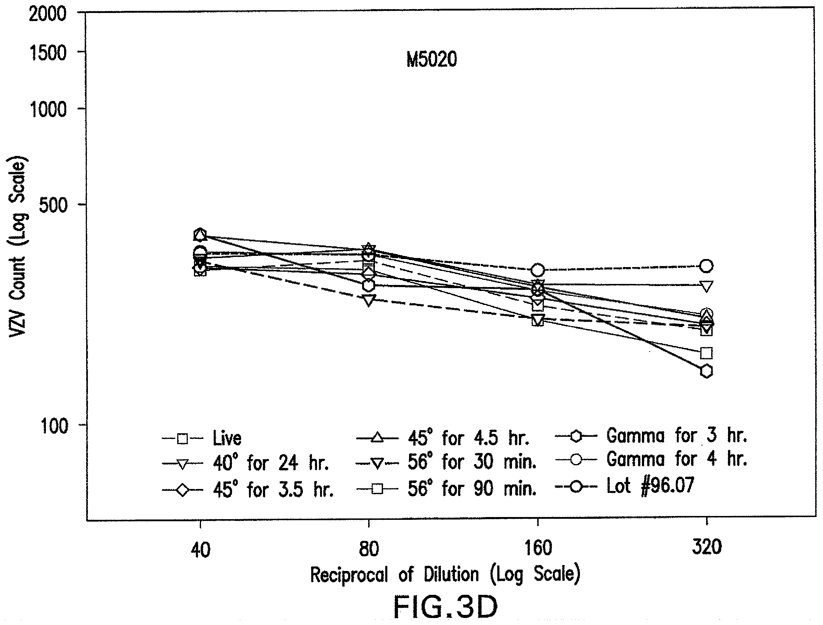

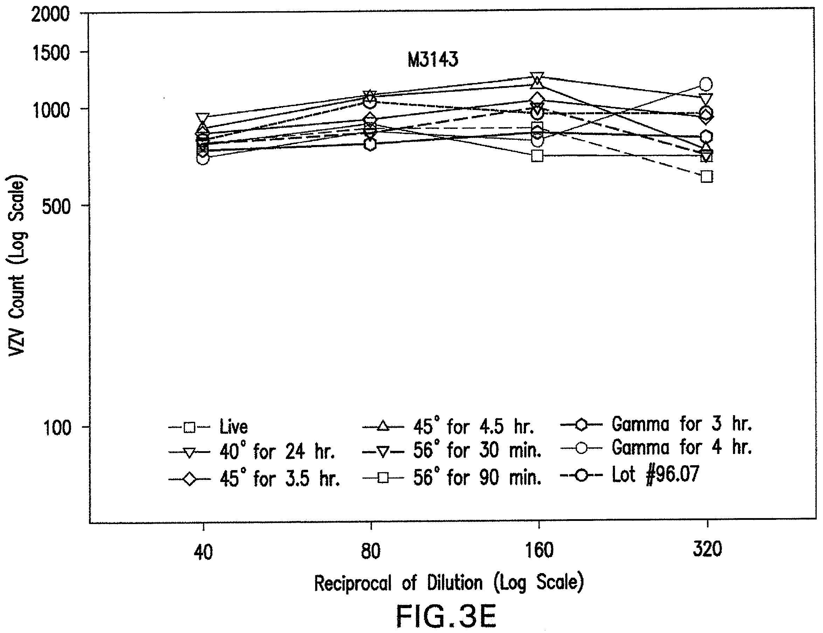

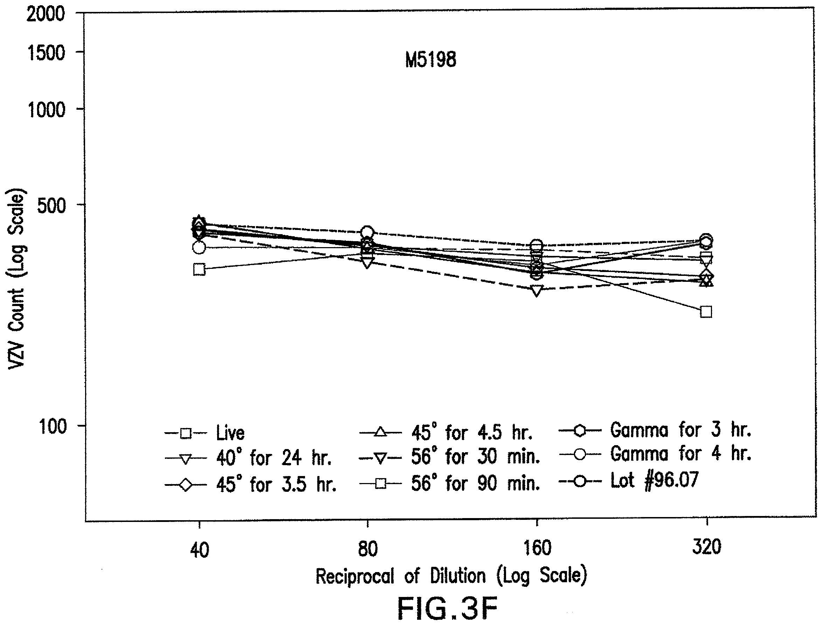

The resulting VZV spot counts (SFC/10⁶ PBMC) are shown in Figures 3A-F. There was no evidence suggesting a difference in VZV response for the inactivated antigen preparations compared with the untreated antigen preparation among the donors tested. There was also no evidence of a significant interaction between preparation (treatment) and response at particular dilution levels. Relative to the live VZV preparation, fold differences in ELISPOT counts for the 8 inactivated VZV preparations, including original VZV Lot #96.07, ranged from 1.15-fold lower (56°C for 90 minutes) to 1.14-fold higher (40°C for 24 hours; Lot #96.07) relative to the live VZV preparation. Relative to the live VZV preparation, none of the 8 inactivated VZV preparations yielded statistically significantly lower ELISPOT counts across the donors tested.

Thus, human ELISPOT testing showed no significant difference in responses using PBMC from a panel of donors, for VZV preparations inactivated by different procedures (40°C, 45°C, 56°C heat-treatment or gamma irradiation of bulk).

### EXAMPLE 6

### Electron Microscopy Analysis of Inactivated VZV Vaccine

EM analyses of heat-treated and gamma irradiated bulks and lyophilized vaccine were performed to provide an additional characterization of the effects of inactivation on inactivated VZV preparations. The analysis method selected was cryo-transmission electron microscopy (cryo-TEM) in vitreous ice to best preserve virus integrity during the EM analysis. Cryo-TEM was performed by NanoImaging Services, Inc (San Diego, CA).

Inactivated VZV samples were prepared for EM analysis to evaluate the effects of inactivation on particle integrity/appearance. Lyophilized samples were rehydrated with 700 µL of sterile distilled water and were not further diluted prior to imaging. Samples were preserved in a thin film of vitreous ice supported on a 2.0x0.5 um C-Flat holey carbon film (Protochips, Inc., Raleigh, NC) on 400 mesh copper grids. Grids were cleaned immediately prior to use in a Solarus plasma cleaner (10 seconds, 25% O₂, 75% Ar). All samples were prepared by applying a drop (∼3 µL) of the sample suspension to the plasma cleaned grid, blotting away with filter paper and immediately processing with vitrification in liquid ethane, using an FEI Vitrobot™ (4C, 95% RH). Grids were stored under liquid nitrogen until transferred to the electron microscope for imaging. Electron microscopy was performed using an FEI Tecnai™ T12 electron microscope (FEI Company, Hillsboro, OR), operating at 120 KeV equipped with an FEI Eagle™ 4K x 4K CC camera (FEI Company). Images of each grid were acquired at multiple scales to assess overall distribution of the specimen. After identifying target areas for imaging at lower magnifications, pairs of higher magnification images were acquired.

Samples were evaluated using a NanoImaging Systems instrument (21K-52K magnification). Untreated lyophilized VZV, lyophilized VZV heat-treated for 77 days at 56°C, and lyophilized VZV irradiated to 25 or 50 kGy using a ⁶⁰Co source were compared.

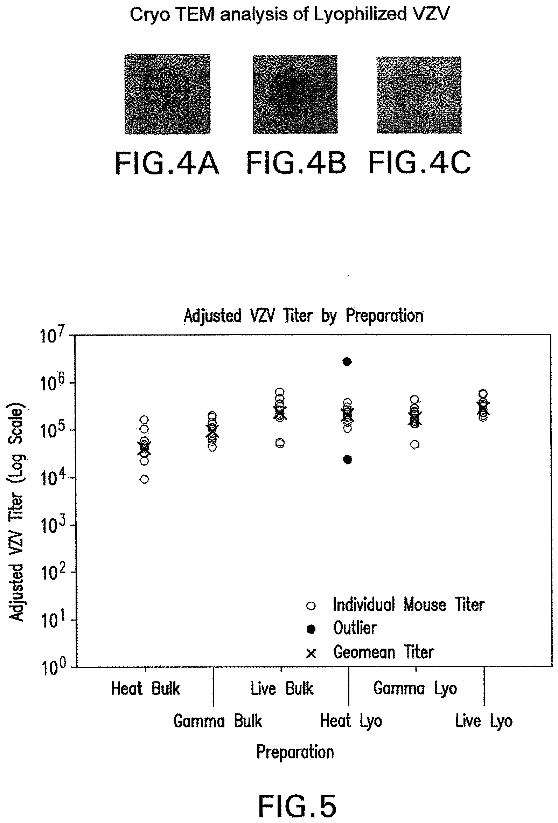

Cryo EM results indicate potential particle effects at high gamma irradiation doses, with a reduction in electron density (perhaps reflecting degradation of viral DNA), but no significant effect of overall particle and protein appearance for lyophilized samples. However, lyophilization may protect from these changes as lyophilized inactivated samples showed a loss of electron density, but particles remained intact at higher doses. (See Figure 4).

### EXAMPLE 7

### Determination of Antigen Reactivity

VZV Ag ELISA assays were performed to determine the antigen (Ag) content of heat or gamma-irradiation inactivated bulk samples or gamma irradiated lyophilized VZV, using a human polyclonal serum and monoclonal antibodies (mAb) to VZV gB, gH, gE as detecting antibodies. Gamma irradiated bulk or lyophilized samples showed no change in antigen reactivity. However, samples heat-treated at 56°C for 90 minutes showed a change in antigen reactivity with the polyclonal, gE and gH monoclonal antibodies.

Additional VZV bulk samples were gamma irradiated at 25kGy and 50kGy to support the use of 50 kGy as an upper exposure for terminal sterilization. These samples were tested in a direct binding antigen ELISA assay to polyclonal anti-VZV serum and mAb to gpE, gB, and gH.

Gamma irradiation of lyophilized VZV vaccine to sterilizing conditions (25-50 kGy) did not detectably affect ELISA reactivity of the antigen with polyclonal serum or gE or gB monoclonal antibodies. Results using the gH mAb were inconclusive due to high background staining. An increased background signal with this monoclonal antibody (and not those to gE or gB) was also observed using an alternate ELISA plate (Maxisorp).

### EXAMPLE 8

### Mouse Immunogenicity

A mouse immunogenicity study was performed to evaluate a set of inactivated VZV (iVZV) preparations generated using various methods for inactivation. Serum samples generated from the study were evaluated in the VZV ELISA assay to determine the relative titer of antibodies to VZV generated by the different inactivated VZV formulations. Two different VZV bulk preparations were generated and evaluated: (1) VZV bulk, heat-treated at 56°C for 90 minutes, and (2) VZV bulk, gamma irradiated with 5,924.7 Grays (Gy) for 3 hours. Two different VZV lyophilized vaccine preparations were also generated and evaluated: (1) VZV Vaccine Lyophilized, heat-treated at 56°C for 50 days, and (2) VZV vaccine lyophilized, gamma irradiated with 25,000 Gy. Live (untreated) VZV bulk and live (untreated) VZV lyophilized vaccine were evaluated for comparison with the iVZV bulk preparations.

BALB/c mice (n=16 for each group) were immunized intraperitoneal (i.p.) with 4.5 VZV antigen units (U)/mouse (9U/mL) on days 0, 14 and 29. Bleeds (sera) were collected on day -1 (pre-bleed) and on days 13, 28 and 43. Day 28 sera (14 days post dose 2) were evaluated in the VZV ELISA for IgG response.

The adjusted VZV titers (VZV titer minus MRC-5 titer) are shown in Figure 5. Pre-bleed sera were tested with the corresponding group from day 28 to determine a titer cut-off for MRC-5 and VZV. Wells of ELISA plates were coated with UV-treated VZV or MRC-5 extract, and then incubated with dilution of the test mouse sera to quantify the relative amounts of antibodies specific for VZV in the mouse sera.

Results indicate that the IgG titer of heat-treated bulk was on average 5.57 fold lower (95% CI = (3.45, 8.99)) and the IgG titer of gamma-irradiated bulk was on average 2.43 fold lower (95% CI = (1.51, 3.93)) relative to untreated bulk. Heat-treated lyophilized titer was on average 1.38 fold lower (95% CI = (1.00, 1.90)) and gamma-irradiated lyophilized titer was on average 1.66 fold lower (95% CI = (1.22, 2.26)) than the untreated lyophilized virus. Antibody titer with gamma-irradiated bulk was 2.29-fold higher (95% CI = (1.54, 3.41)) on average than the titer obtained following immunization with the heat-treated bulk. Antibody titer with gamma-irradiated lyophilized virus was 1.20-fold lower (95% CI = (0.89, 1.61) on average than the titer obtained following immunization with the heat-treated lyophilized preparation. The overall data support gamma irradiation of lyophilized virus as having least impact on immunogenicity in this model.

Results show that antibody (IgG) responses were reduced in mice following immunization with 2 doses of the inactivated preparations compared with the untreated bulks.

### EXAMPLE 9

### Administration of inactivated VZV vaccine to healthy patients aged 50-59.

A gamma -irradiated VZV vaccine was administered to healthy adults 50-to-59 years of age in a randomized, double-blind, 2-part, multicenter study, which was designed to evaluate the safety, tolerability, and immunogenicity of the vaccine. The purpose of this study was to study the safety, tolerability and immunogenicity of vaccine lots inactivated by gamma-irradiation or heat-treatment methods.

A total of 161 healthy individuals 50 to 59 years of age were randomized 2:2:1 to receive either gamma-irradiated VZV vaccine "A", inactivated at 16 to 25 kGy (n=65), heat-treated VZV vaccine (n=63), or placebo (n=33) given as a 4-dose regimen. Each dose was administered approximately 30 days apart. The heat-treated vaccine and the gamma-irradiated vaccine "A" were derived from independent bulk lots but were controlled by targeting similar antigen content.

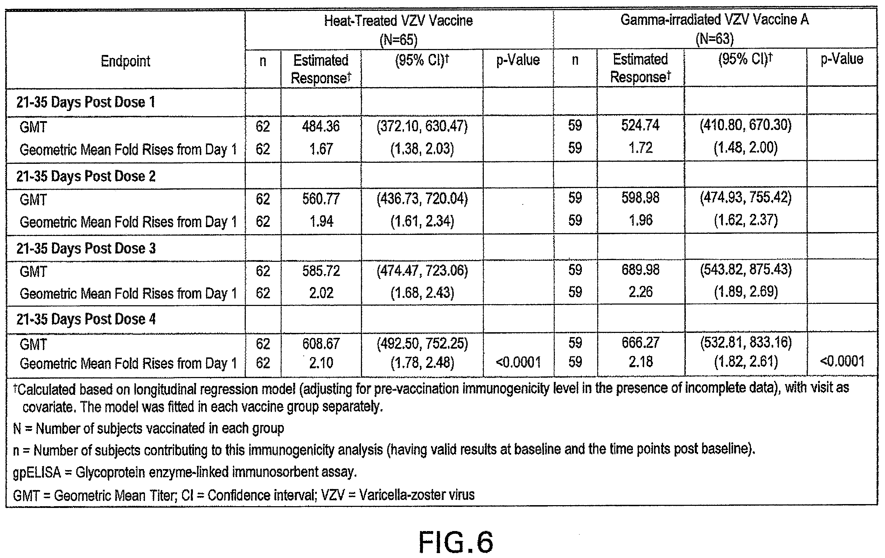

The primary hypothesis was that gamma-irradiated VZV vaccine (A) inactivated at a level of 16 to 25 kGy would elicit an acceptable VZV-specific immune response as measured by gpELISA at 28 days Postdose 4. The secondary hypothesis was that heat-treated VZV vaccine would elicit an acceptable VZV-specific immune response as measured by gpELISA at 28 days Postdose 4. The statistical criterion for success corresponds to the lower bound of the two-sided 95% confidence interval on the geometric mean fold rise (GMFR) in the heat-treated VZV vaccine recipients being >1.0. The immunogenicity results among vaccine recipients in this study are provided in Figure 6.

Results indicate that the success criteria for testing the primary and secondary hypotheses were met, i.e., gamma-irradiated VZV vaccine A and heat-treated VZV vaccine were immunogenic when administered to healthy individuals. Both vaccine groups had safety profiles similar to that of the placebo group.

### EXAMPLE 10

### Administration of VZV vaccine lots inactivated with different levels of gamma irradiation to healthy volunteers aged 50 to 59.

A second study was conducted to provide clinical data on vaccine lots inactivated with a broader range of gamma irradiation. In this study, a total of 129 healthy individuals 50 to 59 years of age were randomized 1:1 to receive either gamma-irradiated VZV vaccine "B", irradiated at 16 to 25 kGy (n=64) or gamma-irradiated VZV vaccine "C", irradiated at 25 to 50 kGy (n=65) given as a 4-dose regimen, each dose administered approximately 30 days apart. Vaccine lots B & C utilized in this study were derived from the same bulk lot and same fill/formulation with identical VZV antigen content but were inactivated at different gamma-irradiation levels.

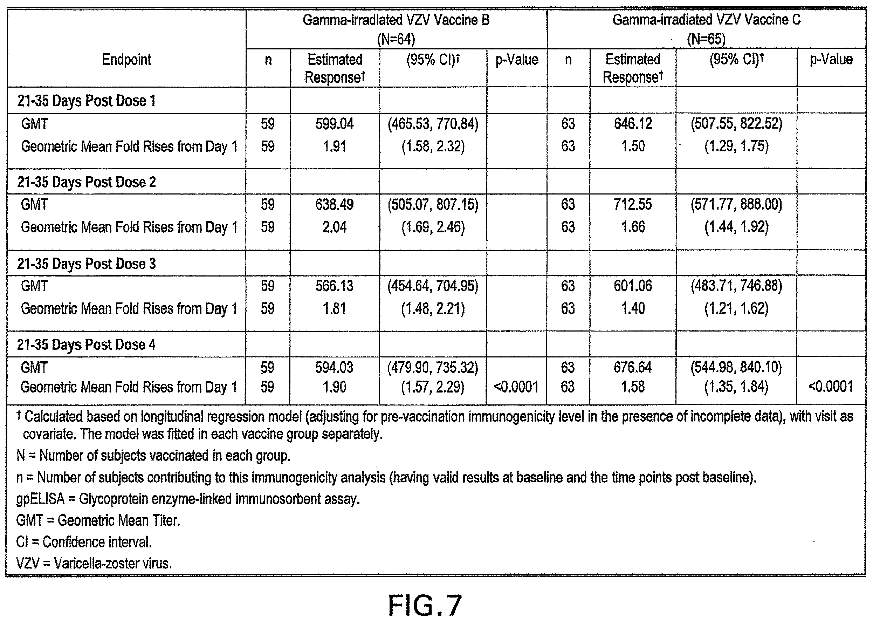

The first primary hypothesis of this study was that gamma-irradiated VZV vaccine (C) inactivated at a level of 25 to 50 kGy would elicit an acceptable VZV-specific immune response as measured by gpELISA at 28 days Postdose 4. The second primary hypothesis was that gamma-irradiated VZV vaccine (B) inactivated at a level of 16 to 25 kGy and matched for antigen content with the vaccine inactivated at 25 to 50 kGy will elicit an acceptable VZV-specific immune response as measured by gpELISA at 28 days Postdose 4. The statistical criteria of success for each hypothesis correspond to the lower bound of the two-sided 95% confidence interval on the GMFR in the gamma-irradiated vaccine recipients being >1.0. The immunogenicity results among vaccine recipients in this study are provided in Figure 7.

Results of this study indicate that the success criteria for testing the primary hypotheses were met, i.e., gamma-irradiated VZV vaccine B (gamma-irradiated 16-25 kGy) and gamma-irradiated VZV vaccine C (gamma-irradiated 25-50 kGy) were immunogenic when administered to healthy individuals. Both vaccine groups had similar safety profiles.

Results of this study also indicate that there was an imbalance of the baseline geometric mean titer (GMT); group B baseline GMT was ∼313, group C baseline GMT was ∼429. Although the fold rise differences were not significant, the difference in baseline titers may account for the fold rise differences observed in the trial. In addition, the analytical data indicates the higher gamma-irradiation dose (>25 kGy) results in a higher antigen degradation.

### FEATURES

1. An inactivated varicella zoster virus (VZV), wherein the infectivity of the VZV is undetectable and wherein the inactivated VZV induces an immune response against VZV when administered to a patient.
2. A pharmaceutical composition comprising a therapeutically effective amount of the VZV of feature 1 and a pharmaceutically acceptable carrier
3. The pharmaceutical composition of feature 2, wherein the infectivity of the inactivated VZV is ≤0.040 plaque-forming units (PFU's)/mL.
4. The pharmaceutical composition of feature 3, wherein the infectivity of the inactivated VZV is ≤0.010 plaque-forming units (PFU's)/mL
5. The pharmaceutical composition of feature 4, wherein the infectivity of the inactivated VZV is ≤0.002 plaque-forming units (PFU's)/mL
6. The pharmaceutical composition of any of features 2-5, wherein the VZV strain is an Oka strain or an Oka strain derivative.
7. The pharmaceutical composition of feature 6, wherein the composition is lyophilized.
8. The pharmaceutical composition of feature 7, wherein the VZV is inactivated by gamma irradiation.
9. The pharmaceutical composition of feature 8, wherein the immune response elicited by the composition is not significantly different than the immune response elicited by a control sample comprising the same amount of the VZV that has not been inactivated.
10. The pharmaceutical composition of feature 9, wherein the immune response elicited by the inactivated VZV differs from the immune response elicited by the control sample by 25% or less.
11. The pharmaceutical composition of any of features 2-5, wherein the infectivity of the VZV is determined by a varicella plaque assay.
12. A method of preparing an inactivated varicella zoster virus (VZV) comprising gamma irradiating a sample comprising a VZV using from about 5 to about 50 kGy of gamma radiation.
13. The method of feature 12, wherein the gamma radiation is provided to the sample by exposing the sample to ⁶⁰Co rays.
14. The method of feature 13, wherein the VZV is the Oka strain or an Oka strain derivative.
15. The method of feature 13, wherein the sample is lyophilized prior to being irradiated.
16. The method of feature 14 or feature 15, wherein the amount of gamma radiation used is about 25 kGy or less.
17. The method of feature 16, wherein the vaccine is a liquid bulk formulation and the amount of gamma radiation used is from about 5 kGy to about 12.5 kGy.
18. The method of feature 15, wherein the amount of gamma radiation used is from about 10 kGy to about 25 kGy.
19. An inactivated VZV produced or obtainable by the method of any of features 12-18.
20. A vaccine comprising a therapeutically effective amount of the inactivated VZV of feature 19 and a pharmaceutically acceptable carrier.
21. A method for the treatment of herpes zoster in a subject, the method comprising administering to the subject a pharmaceutical composition which comprises a therapeutically effective amount of an inactivated varicella zoster virus (VZV) and a pharmaceutically acceptable carrier, wherein the VZV is inactivated with gamma irradiation.
22. The method of feature 21, wherein the route of administration of the pharmaceutical composition is subcutaneous or intramuscular.
23. The method of feature 22, wherein the subject is 50 years of age or older.
24. The method of feature 22 or 23, wherein the subject is immunocompromised.
25. The method of feature 24, wherein the subject has at least one condition selected from the group consisting of: suffering from a hematologic malignancy, undergoing an immunosuppressive therapy, received a hematopoietic stem cell transplant, received a solid organ transplant, infected with HIV, and suffering from an autoimmune disease.
26. The method of feature 22 or 23, wherein the method further comprises allowing a predetermined amount of time to pass, and administering to the subject one or more doses of the pharmaceutical composition.

## Claims

1. An inactivated varicella zoster virus (VZV), wherein the infectivity of the VZV is undetectable and wherein the inactivated VZV induces an immune response against VZV when administered to a patient.

2. A pharmaceutical composition comprising a therapeutically effective amount of the VZV of claim 1 and a pharmaceutically acceptable carrier

3. The pharmaceutical composition of claim 2, wherein the infectivity of the inactivated VZV is ≤0.040 plaque-forming units (PFU's)/mL.

4. The pharmaceutical composition of claim 3, wherein the infectivity of the inactivated VZV is ≤0.010 plaque-forming units (PFU's)/mL

5. The pharmaceutical composition of claim 4, wherein the infectivity of the inactivated VZV is ≤0.002 plaque-forming units (PFU's)/mL

6. The pharmaceutical composition of any of claims 2-5, wherein the VZV strain is an Oka strain or an Oka strain derivative.

7. The pharmaceutical composition of claim 6, wherein the composition is lyophilized.

8. The pharmaceutical composition of claim 7, wherein the VZV is inactivated by gamma irradiation.

9. The pharmaceutical composition of claim 8, wherein the immune response elicited by the composition is not significantly different than the immune response elicited by a control sample comprising the same amount of the VZV that has not been inactivated.

10. The pharmaceutical composition of claim 9, wherein the immune response elicited by the inactivated VZV differs from the immune response elicited by the control sample by 25% or less.

11. The pharmaceutical composition of any of claims 2-5, wherein the infectivity of the VZV is determined by a varicella plaque assay.

12. A method of preparing an inactivated varicella zoster virus (VZV) comprising gamma irradiating a sample comprising a VZV using from about 5 to about 50 kGy of gamma radiation.

13. The method of claim 12, wherein the gamma radiation is provided to the sample by exposing the sample to ⁶⁰Co rays.

14. The method of claim 13, wherein the VZV is the Oka strain or an Oka strain derivative.

15. The method of claim 13, wherein the sample is lyophilized prior to being irradiated.

16. The method of claim 14 or claim 15, wherein the amount of gamma radiation used is about 25 kGy or less.

17. The method of claim 16, wherein the vaccine is a liquid bulk formulation and the amount of gamma radiation used is from about 5 kGy to about 12.5 kGy.

18. The method of claim 15, wherein the amount of gamma radiation used is from about 10 kGy to about 25 kGy.

19. An inactivated VZV produced or obtainable by the method of any of claims 12-18.

20. A vaccine comprising a therapeutically effective amount of the inactivated VZV of claim 19 and a pharmaceutically acceptable carrier.

21. A method for the treatment of herpes zoster in a subject, the method comprising administering to the subject a pharmaceutical composition which comprises a therapeutically effective amount of an inactivated varicella zoster virus (VZV) and a pharmaceutically acceptable carrier, wherein the VZV is inactivated with gamma irradiation.

22. The method of claim 21, wherein the route of administration of the pharmaceutical composition is subcutaneous or intramuscular.

23. The method of claim 22, wherein the subject is 50 years of age or older.

24. The method of claim 22 or 23, wherein the subject is immunocompromised.

25. The method of claim 24, wherein the subject has at least one condition selected from the group consisting of: suffering from a hematologic malignancy, undergoing an immunosuppressive therapy, received a hematopoietic stem cell transplant, received a solid organ transplant, infected with HIV, and suffering from an autoimmune disease.

26. The method of claim 22 or 23, wherein the method further comprises allowing a predetermined amount of time to pass, and administering to the subject one or more doses of the pharmaceutical composition.
